# EUROPEAN PATENT SPECIFICATION

(11) **EP 2 957 262 B1**
(45) Date of publication and mention of the grant of the patent: **19.04.2017**
(21) Application number: 15172505.8
(22) Date of filing: 17.06.2015
(51) Int. Cl.: A61F 2/16

(54) **INTRAOCULAR LENS INJECTION INSTRUMENT**
EINSPRITZINSTRUMENTE FÜR INTRAOKULARLINSE
INSTRUMENT D'INJECTION DE LENTILLE INTRAOCULAIRE

(30) Priority: 19.06.2014 JP 2014126523
(43) Date of publication of application: 23.12.2015
(73) Proprietor: NIDEK CO., LTD, Gamagori-shi Aichi 443-0038 (JP)
(72) Inventor: NAGASAKA, Shinji, Aichi, 443-0038 (JP); NATSUME, Akiyoshi, Aichi, 443-0038 (JP); INOUE, Takanori, Aichi, 443-0038 (JP)
(74) Representative: Prüfer & Partner mbB Patentanwälte · Rechtsanwälte

(56) References cited:
- EP-A1- 2 074 961
- EP-A1- 2 343 029
- US-A1- 2011 224 677
- US-B1- 6 447 520
- US-B2- 7 131 976

## Description

### BACKGROUND OF THE INVENTION

### Field of the Invention

The present disclosure relates to an intraocular lens injection instrument for injecting an intraocular lens into a patient's eye.

### Related Art

Heretofore, as one method of cataract surgery, a generally used method is to extract a lens and to inject a foldable soft intraocular lens (IOL) as a substitute for the lens into an eye. Further, another example is also known as injecting the IOL anterior to the lens in order to correct refractive power of an eye. Injection of the IOL into an eye is usually performed by use of an intraocular lens injection instrument called as an injector.

When the IOL is to be injected into the eye by the injector, the IOL loaded on a setting part of the injector is pushed by a rod-like push member (plunger) and further pushed toward a tip end of the tapered injector, so that the IOL is folded into a tiny piece along an inner wall shape of the injector (see Patent Documents 1 and 2, for example).

Patent Document 3 discloses an intraocular lens injection instrument for injecting an intraocular lens corresponding to the preamble of claim 1 of the present application.

Patent Document 4 discloses an insertion device for intraocular lens including a push rod for pushing and inserting the lens into the eye, and a posture control member disengagably engaged with a tip end portion of the push rod. The posture control member prevents deflection of the push rod from a center axis. The engagement between the posture control member and the push rod is broken when the push rod arrives at a predetermined position during a step of advancing the push rod.

### Related Art Documents

### Patent Documents

Patent Document 1: Re-publication of International Publication No. WO2011/048631A1
Patent Document 2: Re-publication of International Publication No. WO2010/064275A1
Patent Document 3: EP 2 343 029 A1
Patent Document 4: US 7 131 976 B2

### SUMMARY OF INVENTION

### Problems to be Solved by the Invention

As for the above mentioned injector, for folding the IOL into a desirable tiny piece, it is necessary to precisely control a push-out direction of the push member by centering the push member. For example, a pair of protrusions symmetrically formed are provided (such as a pair of guide rails in the Patent Document 2) and the push member is positioned in between this pair of protrusions for centering.

However, in a conventional injector, such a configuration for centering a push member sometimes obstructs folding the IOL into a desirable shape. If at least one of centering of the push member and a folded positon of the IOL becomes unstable, there is a possibility that the IOL is difficult to be properly injected into the eye.

In response to this, the present disclosure has been made for solving the above problem and has a purpose to provide an intraocular lens injection instrument realizing stable centering of a push member and enabling stabilization of a folded position of an intraocular lens.

### Means of Solving the Problems

The above mentioned problem is solved by the intraocular lens injection instrument according to claim 1 of the present application.

### Effects of the Invention

According to the intraocular lens injection instrument of the present disclosure, stable centering of a push member is realized and a folded position of an intraocular lens can be stabilized.

### BRIEF DESCRIPTION OF THE DRAWINGS

FIG. 1 is an external schematic view of an intraocular lens injection instrument;
FIG. 2 is a cross sectional view taken along a line A-A in FIG. 1;
FIG. 3 is a cross sectional view taken along a line B-B in FIG. 1;
FIG. 4 is an external perspective view of a top plate;
FIG. 5 is a front view of the top plate; FIG. 6 is an external perspective view of a plunger;
FIG. 7 is a plan view of an intraocular lens;
FIG. 8 is a right-side view of the intraocular lens;
FIG. 9 is an explanatory view for explaining a method for injecting the intraocular lens by use of the intraocular lens injection instrument;
FIG. 10 is a cross sectional view taken along a line C-C in FIG. 9;
FIG. 11 is a cross sectional view taken along a line D-D in FIG. 9;
FIG. 12 is an explanatory view for explaining the method for injecting the intraocular lens by use of the intraocular lens injection instrument;
FIG. 13 is a cross sectional view taken along a line E-E in FIG. 12;
FIG. 14 is a cross sectional view taken along a line F-F in FIG. 12;
FIG. 15 is a cross sectional view taken along a line G-G in FIG. 12; and
FIG. 16 is a partial enlarged view of a top plate in a modified embodiment.

### DESCRIPTION OF EMBODIMENTS

A detailed description of an embodiment according to the present disclosure will now be given with reference to the accompanying drawings.

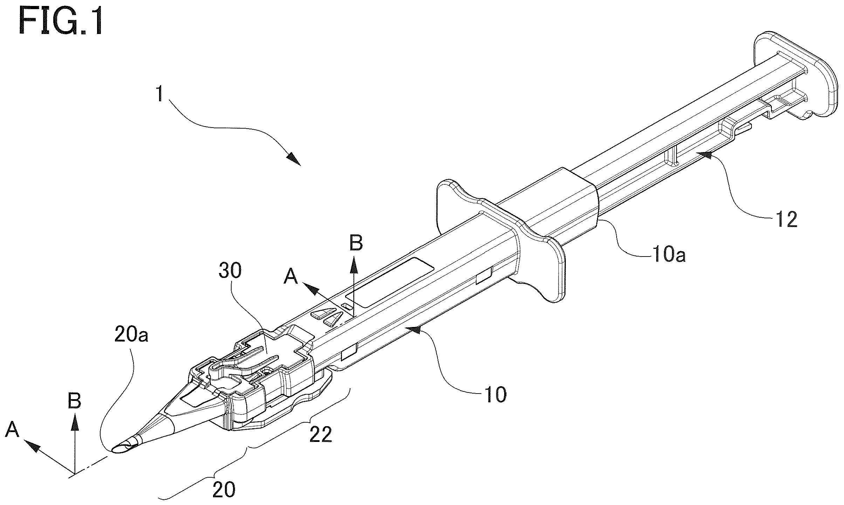

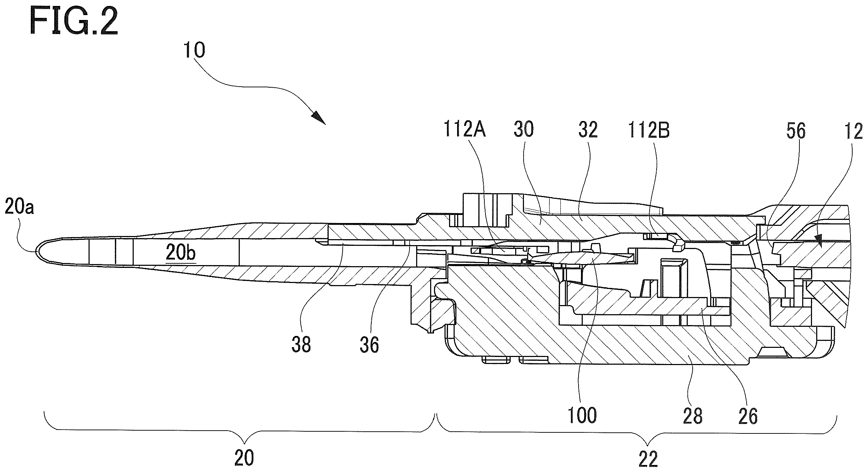

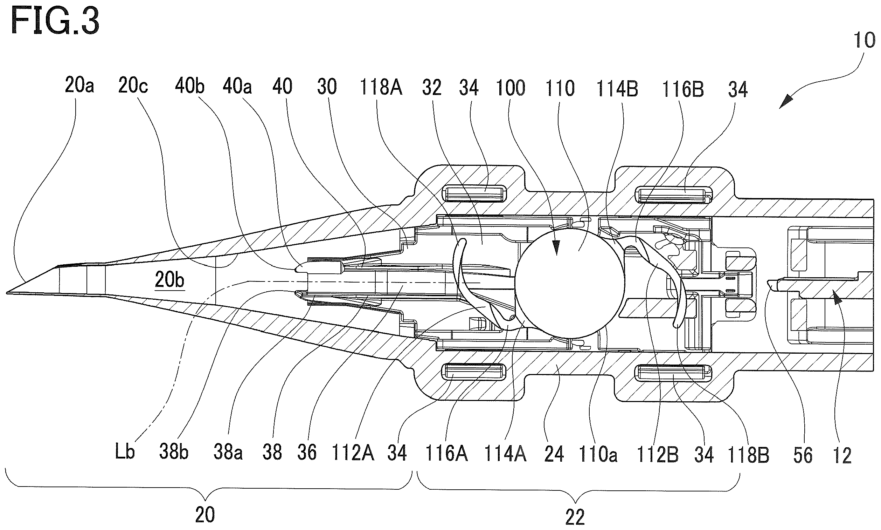

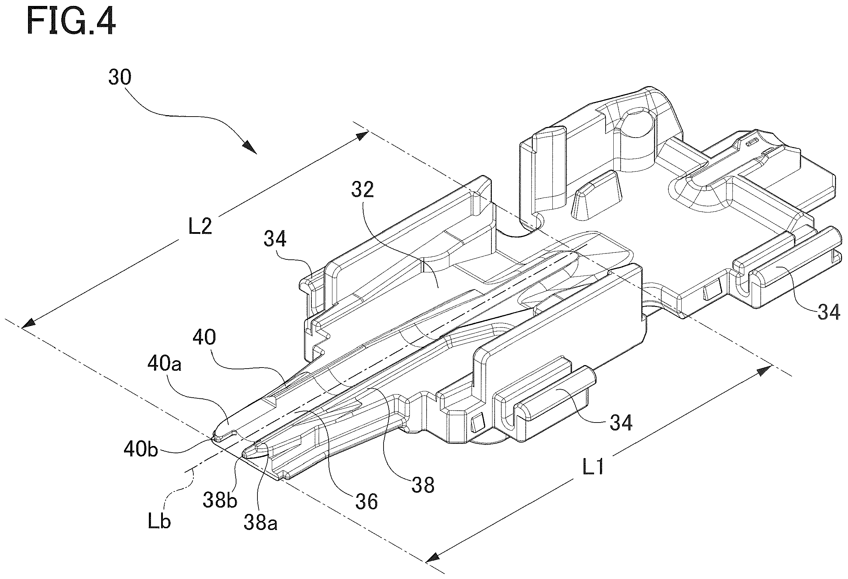

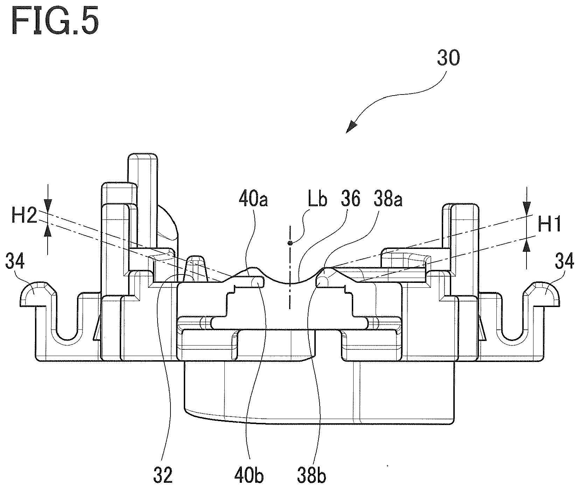

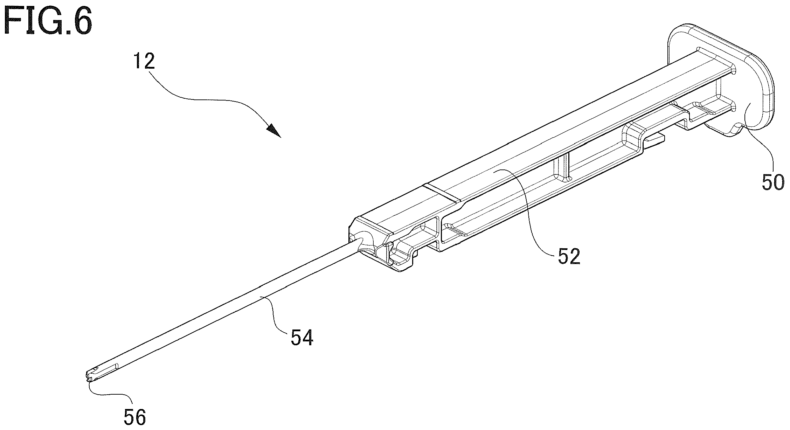

Firstly, a configuration of an injector 1 as an intraocular lens injection instrument is explained. FIG. 1 is an external schematic view of the injector 1. FIG. 2 is a cross sectional view taken along a line A-A in FIG. 1 (a sectional view seen from a side surface direction of the injector 1 when the injector 1 is vertically cut along a longitudinal direction of the injector 1 in FIG. 1. FIG. 3 is a cross sectional view taken along a line B-B in FIG. 1 (a sectional view seen from a lower surface direction when the injector is horizontally cut along the longitudinal direction of the injector 1 in FIG. 1). FIG. 4 is an external perspective view of a top plate 30. FIG. 5 is a front view of the top plate 30. FIG. 6 is an external perspective view of a plunger 12.

As shown in FIGs. 1 to 3, the injector 1 includes a cylinder body (hereinafter, referred as a "body") 10, the plunger 12, and others. The body 10 includes an injection part 20, a setting part 22, and others. This type of injector 1 is, for example, formed by injection molding using resin material or the like. Instead, the injector 1 may be formed as a cutting-processed product made of resin by cutting or machining.

The injection part 20 of the body 10 is formed in a cylindrical hollow shape with a passage 20b of which an inner diameter gradually decreases toward a tip end 20a in the section shown in FIG. 3. The tip end 20a of the injection part 20 is formed with a cutout (bevel) for feeding an intraocular lens (IOL) 100 outside. As explained in detail below, the IOL 100 passing the injection part 20 is folded into a tiny piece along an inner wall surface 20c of the injection part 20, and then fed out from the bevel of the tip end 20a, so that the IOL 100 is injected into an eye.

The setting part 22 of the body 10 is formed in an upstream side (rear end side) of the injection part 20 in the push-out direction of the plunger 12. The setting part 22 is formed inside with a space (clearance) for accommodating the IOL 100 before start of pushing the IOL 100 with the plunger 12. The setting part 22 of the present embodiment includes a setting part main body 24, a holding member 26, a holding member 28, the top plate 30, and others. In this setting part 22, the IOL 100 to be pushed out by the plunger 12 is held in position inside the setting part main body 24 by the holding members 26 and 28.

The top plate 30 also constitutes a part of the injection part 20 as a cover member positioned on a top side of the injection part 20 and the setting part 22. The top plate 30 of the present embodiment is provided with a plate portion 32, engaging portions 34, a guide portion 36, a distal-end side protrusion 38 (first protrusion), a root side protrusion 40 (second protrusion), and others as shown in FIGs, 4 and 5. The engaging portions 34 are engaged with the setting part main body 24. The top plate 30 may be formed by such as injection molding using resin material (for example, polypropylene).

The guide portion 36 is formed on the plate portion 32. As one example, the guide portion 36 of the present embodiment is a groove extending from an almost central portion of the plate portion 32 to the injection part 20. This groove-like guide portion 36 is formed such that its axial line Lb coincides with the push-out direction of the plunger 12 so as to guide the push-out direction of the plunger 12.

The distal-end side protrusion 38 constitutes one side wall surface of the guide portion 36 (a forward side of FIG. 4) and protrudes from the plate portion 32 to a side on which the IOL 100 is to be positioned (an upper side in FIG. 4).

The root side protrusion 40 constitutes the other side wall surface (a rear side of FIG. 4) and protrudes from the plate portion 32 to a side on which the IOL 100 is to be positioned (the upper side in FIG. 4). Specifically, the distal-end side protrusion 38 is placed on one side of the guide portion 36 and the root side protrusion 40 is placed on the other side opposite to the distal-end side protrusion 38 so that the guide portion 36 is held from right and left by the distal-end side protrusion 38 and the root side protrusion 40.

The shape of the distal-end side protrusion 38 and the shape of the root side protrusion 40 are asymmetry. To be specific, in the present embodiment, an end portion 38a of the distal-end side protrusion 38 positioned closer to the tip end 20a of the injection part 20 is formed higher than an end portion 40a of the root side protrusion 40 positioned closer to the tip end 20a of the injection part 20. In other words, the length (height) of the end portion 38a in a vertical direction relative to a plate surface of the plate portion 32 is made longer than the length of the end portion 40a in the vertical direction relative to the plate surface of the plate portion 32. To be more specific, the end portion 40a of the root side protrusion 40 is formed as a cutout. Then, in this manner, a height H1 (protruding amount) of the end portion 38a of the distal-end side protrusion 38 from the plate portion 32 is larger than a height H2 (protruding amount) of the end portion 40a of the root side portion 40 from the plate portion 32. As one example, the height H2 is determined to be 60% to 80% of the height H1.

Then, the end portion 40a of the root side protrusion 40 is formed in a position where a root portion 116B of a rear support part 112B will be placed when an optical part 110 comes to be folded by contact with the inner wall surface 20c of the injection part 20 during injection of the IOL 100 with the plunger 12.

In the example shown in FIG. 4, a length L1 of the distal-end side protrusion 38 in the push-out direction (a forward and backward direction) and a length L2 of the root side protrusion 40 in the push-out direction is made to be equal. Herein, the length L1 is the one from a basal end of the guide portion 36 to a foremost end 38b of the distal-end side protrusion 38. Further, the length L2 is the one from the basal end of the guide portion 36 to a foremost end 40b of the root side protrusion 40.

The plunger 12 is a push member configured to push an outer circumferential portion (an outer rim) 110a of the optical part 110 of the IOL 100 to fold the IOL 100 inside the injection part 20 into a tiny piece and then to push out the folded IOL 100 from the tip end 20a of the injection part 20. As shown in FIG. 6, the plunger 12 of the present embodiment is provided with a press part 50, a basal part 52, a push rod 54, a tip portion 56, and others. The press part 50 is a part to be pressed by an operator who uses the injector 1. The basal part 52 is a part connected to the press part 50. The push rod 54 is a part connected to the basal part 52. The tip portion 56 is a part formed on a tip end of the push rod 54. The plunger 12 with this configuration is inserted in the body 10 in a movable manner in an axial direction of the plunger 12 inside a passage extending from a basal end portion 10a of the body 10 to the tip end 20a of the injection part 20.

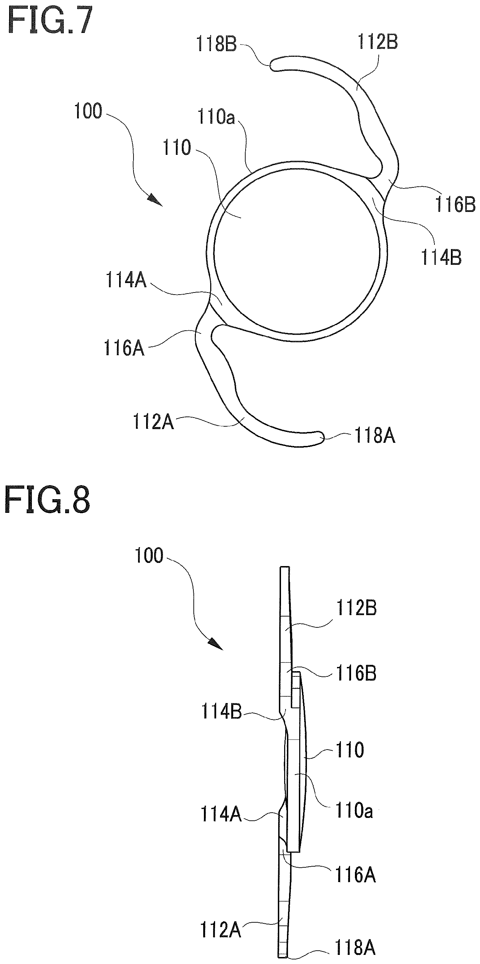

Next, the intraocular lens (IOL) 100 to be used in the present embodiment is explained. FIG. 7 is a plan view of the IOL 100. FIG. 8 is a right-side view of the IOL 100.

As shown in FIGs. 7 and 8, the IOL 100 used in the present embodiment is a one-piece IOL in which the optical part 110 and a pair of support parts including a front support part 112A and the rear support part 112B are integrally formed of flexible material.

The optical part 110 is formed in a disk-like shape. The front support part 112A and the rear support part 112B are formed in point symmetrical positions with respect to a center of the optical part 110 as a reference point on the outer circumferential portion 110a of the optical part 110. In the front support part 112A, a root portion 116A is connected to the outer circumferential portion 110a of the optical part 110 via a connection part 114A, and a distal end 118A is open (the distal end 118A is a free end). In the rear support part 112B, a root portion 116B is connected to the outer circumferential portion 110a of the optical part 110 via a connection part 114B, and a distal end 118B is open.

As mentioned in detail below, in pushing out the IOL 100 with the plunger 12, the rear support part 112B positioned on a side closer to the plunger 12 is folded inwardly toward the outer circumferential portion 110a of the optical part 110. At this time, the distal end 118B of the rear support part 112B is placed on a side closer to the distal-end side protrusion 38 than the axial line of the plunger 12, and the root portion 116B of the rear support part 112B is placed on a side closer to the root side protrusion 40 than the axial line of the plunger 12.

As an operation of an intraocular lens injection instrument, a method of injecting the IOL 100 in an eye by use of the injector 1 is now explained.

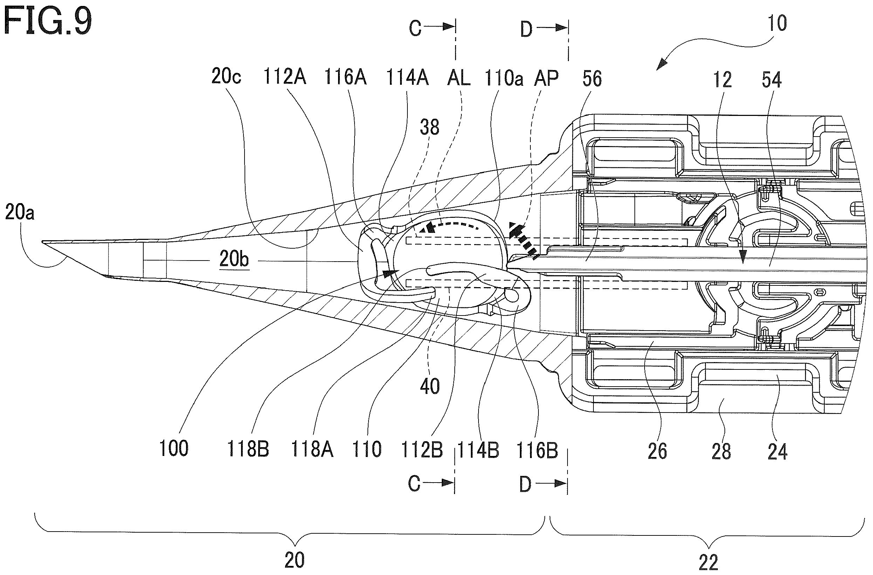

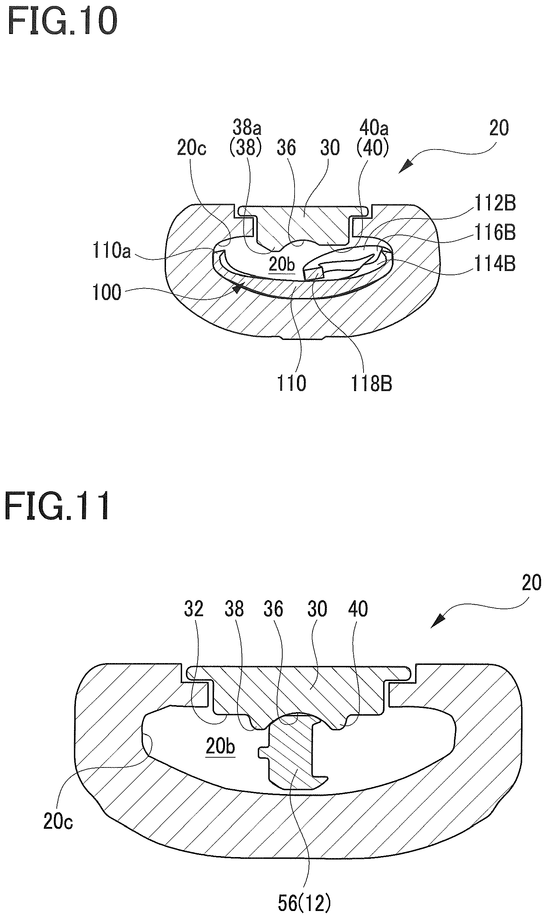

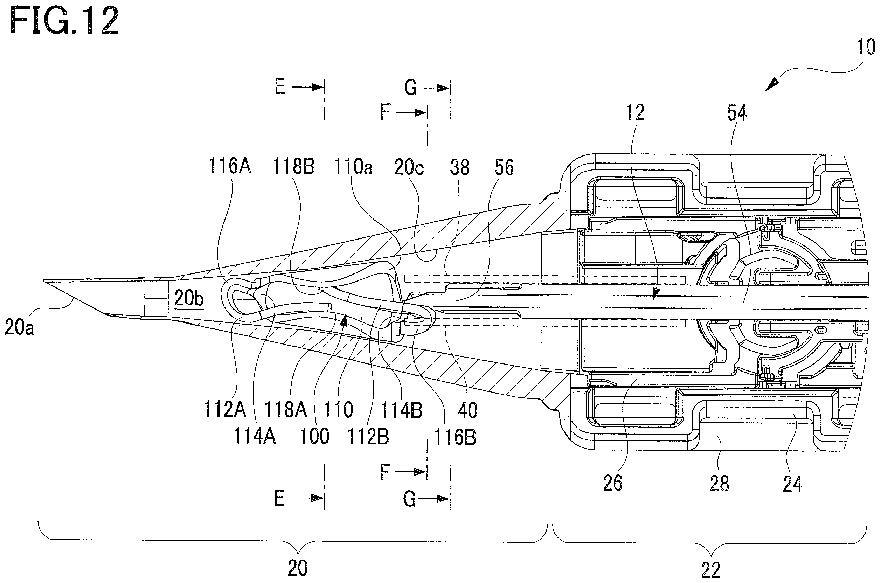

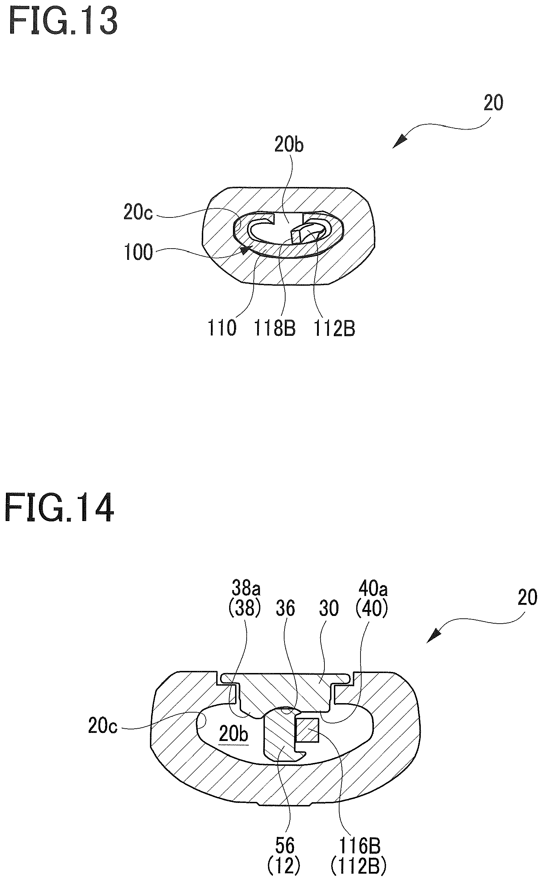

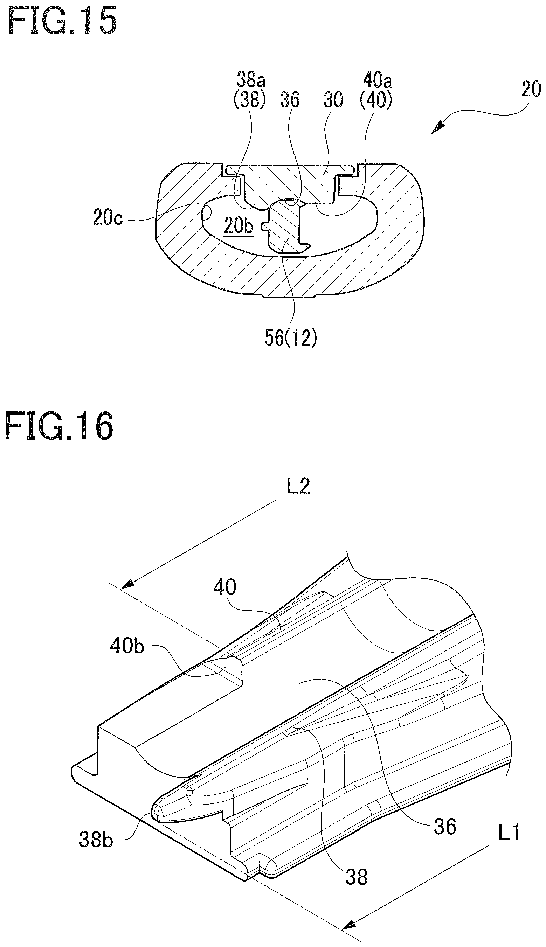

FIGs. 9 and 12 are explanatory views for explaining a method of injecting an intraocular lens using an intraocular lens injection instrument, each corresponding to a sectional view seen from an upper surface direction when the injector 1 is horizontally cut along the longitudinal direction of the injector 1 in Fig. 1. In FIGs. 9 and 12, the positions where the distal-end side protrusion 38 and the root side protrusion 40 of the top plate 30 should be present are indicated with dotted lines. FIG. 10 is a sectional view taken along a line C-C in FIG. 9, and FIG. 11 is a sectional view taken along a line D-D in FIG. 9. Further, FIG. 13 is a sectional view taken along a line E-E in FIG. 12, FIG. 14 is a sectional view taken along a line F-F in FIG. 12, and FIG. 15 is a sectional view taken along a line G-G in FIG. 12.

From a state shown in FIG. 3, an operator operates the injector 1 to push the plunger 12 to move forward the rear support part 112B positioned on the side closer to the plunger 12 of the pair of support parts 112A and 112B of the IOL 100. Thus, as shown in FIG. 9, the root portion 116B of the rear support part 112B is curved and the rear support part 112B is folded inwardly toward the outer circumferential portion 110a of the optical part 110.

As a result, the rear support part 112B is placed in the clearance closer to the top plate 30 (the guide portion 36) relative to the optical part 110. Further, the distal end portion 118B of the rear support part 112B facing toward the tip end 20a of the injection part 20 is positioned more inward than the outer circumferential portion 110a of the optical part 110. Moreover, while the distal end portion 118B of the rear support part 112B is placed on the side closer to the distal-end side protrusion 38, the root portion 116B of the rear support part 112B is placed on the side closer to the root side protrusion 40.

The optical part 110 of the IOL 100 comes to contact with the inner wall surface 20c of the injection part 20 and starts to be folded as valley-folded toward a rear side of FIG. 9. The front support part 112A of the IOL 100 being in contact with the inner wall surface 20c of the injection part 20 starts to be folded such that the root portion 116A is gradually curved to direct the distal end portion 118A toward the plunger 12. Herein, since the inner wall surface 20c of the injection part 20 is applied with coating for smooth movement of the front support part 112A, the IOL 100 hardly rotates. Accordingly, the front support part 112A is more surely folded.

As shown in FIG. 10, the distal end portion 118B of the rear support part 112B of the IOL 100 reaches a position where the end portion 40a of the root side protrusion 40 is placed in the push-out direction of the plunger 12.

Further as shown in FIG. 11, the tip portion 56 of the plunger 12 is located inside the guide portion 36 of the top plate 30. Thus, the plunger 12 is guided its push-out direction by the guide portion 36 of the top plate 30. Accordingly, centering of the plunger 12 is realized in a direction perpendicular to the push-out direction of the plunger 12 and a horizontal direction relative to a surface of the optical part 110 prior to folding (in an upper and lower direction in FIG. 9 and in a left and right direction in FIGs. 10 and 11, heretofore referred simply as "a horizontal direction relative to the surface of the optical part 110").

Like the above conventional arts, it is now assumed what if the height H1 of the end portion 38a of the distal-end side protrusion 38 and the height H2 of the end portion 40a of the root side protrusion 40 are the same and the distal-end side protrusion 38 and the root side protrusion 40 are formed symmetry, while the end portion 40a of the root side protrusion 40 has no cutout.

In this case, when the operator then further pushes the plunger 12 to move the outer circumferential portion 110a of the optical part 110 of the IOL 100 from the state shown in FIG. 9, the optical part 110 of the IOL 100 is further folded, leading to increase in resilient force (elastic force) generated on the root portion 116B of the rear support part 112B.

As a consequence, due to an influence of the resilient force generated on the root portion 116B of the rear support part 112B, the IOL 100 pushed by the plunger 12 attempts to rotate in a left direction (counterclockwise) in the figure as indicated with a dotted arrow AL in FIG. 9. As a result, the tip portion 56 of the plunger 12 trying to push out the IOL 100 tends to come closer to a direction indicated with a dotted arrow AP in FIG. 9, i.e., a direction in which the distal-end side protrusion 38 is present, so that the plunger 12 could cross over the distal-end side protrusion 38 and deviate from the guide portion 36. Consequently, there is a possibility that centering of the plunger 12 in the horizontal direction relative to the surface of the optical part 110 cannot be realized.

Further, the root portion 116B of the rear support part 112B could come to firmly contact with the end portion 40a of the root side protrusion 40. This could cause large frictional force between the end portion 40a of the root side protrusion 40 and the root portion 116B of the rear support part 112B, and the rear support part 112B tries to extend rearward (a direction opposite to a direction to which the IOL 100 is to be pushed out). Accordingly, the folded position of the rear support part 112B becomes unstable.

On the contrary, in the present embodiment, as shown in FIG. 5, the distal-end side protrusion 38 and the root side protrusion 40 are shaped asymmetry. To be specific, in the injector 1 exemplified in the present embodiment, the height H2 of the end portion 40a of the root side protrusion 40 is made shorter than the height H1 of the end portion 38a of the distal-end side protrusion 38. Owing to this configuration, firm centering of the plunger 12 in the horizontal direction relative to the surface of the optical part 110 can be achieved, and the folded position of the IOL 100 (especially the rear support part 112B in the present embodiment) can be stabilized with the following reasons.

When the operator further pushes the outer circumferential portion 110a of the optical part 110 of the IOL 100 with the plunger 12 after the state shown in FIG. 9, the IOL 100 comes into a state shown in FIG. 12. Then, as shown in FIG. 12, while being in contact with the inner wall surface 20c of the injection part 20, the optical part 110 of the IOL 100 is valley-folded into a tiny piece toward the rear side of FIG. 12. Further, the front support part 112A of the IOL 100 comes to contact with the inner wall surface 20c of the injection part 20 and further, is curved its root portion 116A and folded such that the distal end portion 118A faces toward the plunger 12.

Moreover, as shown in FIG. 12, the rear support part 112B maintains its folded position inside the outer circumferential portion 110a of the optical part 110.

At this time, as shown in FIG. 13, the distal end portion 118B of the rear support part 112B of the IOL 100 has already got out of the position where the root side protrusion 40 is formed.

On the other hand, as shown in FIG. 14, the root portion 116B of the rear support part 112B of the IOL 100 is located in a position where the end portion 40a of the root side protrusion 40 is formed in the push-out direction of the plunger 12. In this state, as shown in FIG. 5, the height H2 of the end portion 40a of the root side protrusion 40 is formed to be shorter than the height H1 of the end portion 38a of the distal-end side protrusion 38 (namely, the end portion 40a of the root side protrusion 40 is formed low). Therefore, as shown in FIG. 14, it is less possible that the root portion 116B of the rear support part 112B folded inwardly in the IOL 100 comes to firmly contact with the end portion 40a of the root side protrusion 40.

Accordingly, between the end portion 40a of the root side protrusion 40 and the root portion 116B of the rear support part 112B, there is no frictional force or only a small frictional force is generated. Therefore, the folded rear support part 112B is prevented from extending rearward of the optical part 110 (to a direction opposite to a direction to which the IOL 100 is pushed out). Accordingly, the folded position of the rear support part 112B is stabilized.

As shown in FIG. 5, the height H1 of the end portion 38a of the distal-end side protrusion 38 is higher than the height H2 of the end portion 40a of the root side protrusion 40. In other words, the end portion 38a of the distal-end side protrusion 38 placed in a position where the plunger 12 is likely to come closer is formed high. Thus, even when the optical part 110 of the IOL 100 is folded to cause increase in the resilient force generated on the root portion 116B of the rear support part 112B, and the plunger 12 is likely to come closer to the distal-end side protrusion 38, the plunger 12 hardly gets out of the guide portion 36 by the contact with the end portion 38a of the distal-end side protrusion 38 as shown in FIGs. 14 and 15. Accordingly, centering of the plunger 12 in a radial direction of the optical part 110 of the IOL 100 is realized.

After that, the operator further pushes the outer circumferential portion 110a of the optical part 110 of the IOL 100 with the plunger 12. Then, the front support part 112A and the rear support part 112B are folded from the root portions 116A and 116B so that the IOL 100 is pushed out of the injection part 20 from the tip end 20a while the IOL 100 is kept valley-folded in a tiny piece, and thereby the IOL 100 is moved into the eye. In this manner, the IOL 100 is injected in the eye.

As a modified embodiment, as shown in FIG. 16, the root side protrusion 40 may be formed shorter than the distal-end side protrusion 38 in a direction to which the IOL 100 is pushed out. Specifically, the length L2 of the root side protrusion 40 in the push-out direction may be set shorter than the length L1 of the distal-end side protrusion 38. Then, in this manner, the foremost end 38b of the distal-end side protrusion 38 may be formed closer to the tip end 20a side of the injection part 20 than the foremost end 40b of the root side protrusion 40. In addition, the foremost end 40b of the root side protrusion 40 is preferably formed on an upstream side where the root portion 116B of the rear support part 112B is positioned in the push-out direction of the plunger 12 when the optical part 110 is folded in pushing out the IOL 100 by the plunger 12.

As one alternative, the end portion 38a of the distal-end side protrusion 38 may be formed higher than the end portion 40a of the root side protrusion 40 and the root side protrusion 40 may be made shorter than the distal-end side protrusion 38.

Further, when the rear support part 112B is folded at the root portion 116B, the rear support part 112B may be positioned on a side of the holding members 26 and 28, which is an opposite side to the top plate 30 side with respect to the optical part 110. In this case, the IOL 100 is mountain-folded.

As mentioned above, according to the present disclosure, in the injector 1, the shape of the distal-end side protrusion 38 and the shape of the root side protrusion 40 are asymmetry. Accordingly, the injector 1 of the above mentioned embodiment can realize firm centering of the plunger 12 and can stabilize the folded position of the IOL 100.

To be specific, in the above embodiment, the height H1 of the end portion 38a of the distal-end side protrusion 38 is higher than the height H2 of the end portion 40a of the root side protrusion 40. In this manner, the end portion 38a of the distal-end side protrusion 38 to which the plunger 12 is likely to come closer is arranged high. Thus, the plunger 12 hardly gets out of the guide portion 36 due to contact with the end portion 38a of the distal-end side protrusion 38 even if the plunger 12 tends to come closer to the distal-end side protrusion 38. As a result, the firm centering of the plunger 12 in the horizontal direction relative to the surface of the optical part 110 is realized.

Further, the end portion 40a of the root side protrusion 40 having the lower height than the end portion 38a of the distal-end side protrusion 38 is formed to be in a position corresponding to a position where the root portion 116B of the rear support part 112B is placed when the optical part 110 of the IOL 100 is to be folded in pushing out the IOL 100 with the plunger 12. Thus, when the optical part 110 is folded, it is possible to eliminate or reduce the frictional force between the end portion 40a of the root side protrusion 40 and the root portion 116B of the rear support part 112B of the IOL 100. Hence, when the optical part 110 is folded, the rear support part 112B folded inwardly in the optical part 110 of the IOL 100 is prevented from extending rearward. Accordingly, the folded position of the rear support part 112B can be stabilized.

Further, in the push-out direction of the IOL 100, the root side protrusion 40 is formed shorter than the distal-end side protrusion 38, so that it is possible to eliminate or reduce the frictional force between the root side protrusion 40 and the root portion 116B of the rear support part 112B. Accordingly, the folded position of the rear support part 112B is further surely stabilized.

Further, the guide portion 36, the distal-end side protrusion 38, and the root side protrusion 40 are formed in the top plate 30 as a cover member constituting a part of the injection part 20. Since the guide portion 36, the distal-end side protrusion 38, and the root side protrusion 40 are integrally formed on the top plate 30, dimensional precision can be enhanced. Therefore, firm centering of the plunger 12 in the radial direction of the optical part 110 of the IOL 100 can be further surely realized.

The above mentioned embodiment is merely an exemplification of the present disclosure, and the present disclosure is not limited to the embodiment. The present disclosure may be applied with various changes and modifications without departing from the scope of its subject matter. For example, in the above embodiment and the modified embodiment, at least either one of the heights or the lengths of the distal-end side protrusion 38 and the root side protrusion 40 are made to be different so that the distal-end side protrusion 38 and the root side protrusion 40 are asymmetry. Alternatively, it is possible to differentiate elements other than the heights and lengths (such as a width and a frictional force on a surface). One example is conceived in such a way that a width of the distal-end side protrusion 38 is made thick to prevent deformation of the distal-end side protrusion 38 and that a width of the root side protrusion 40 is made thin to facilitate deformation of the root side protrusion 40. In this case, the plunger 12 is hardly deformed in a direction of the distal-end side protrusion 38, improving a centering precision. Moreover, the root portion 116B of the rear support part 112B is hardly applied with frictional force from the root side protrusion 40 even if the root portion 116B comes to contact with the root side protrusion 40 which is easy to be deformed.

The technique exemplified in the above embodiment may be applied to a preset type of intraocular lens injection instrument in which the IOL 100 is loaded inside in advance, and also may be applied to an intraocular lens injection instrument provided without being loaded with the IOL 100 in advance. The IOL 100 illustrated in the above embodiment is a one-piece IOL in which the optical part 110 and the support parts 112 are integrally formed. Alternatively, the technique exemplified in the above embodiment may be applied to an intraocular lens injection instrument for injecting an IOL other than a one-piece type (for example, a three-piece IOL and others) into an eye. The root portions 116A and 116B of the IOL 100 may be directly connected to an outer circumferential part of the optical part 110 without interposing the connection parts 114A and 114B.

A shape of the IOL 100 may also be varied. For example, even when the shape of the IOL 100 is changed, the IOL 100 being pushed out is sometimes shaped to be asymmetry with respect to a pushing axis as a center as similar to the above embodiment. Also in this case, two protrusions 38 and 40 may be formed asymmetry such that one of those protrusions 38 and 40 that receives larger force from the asymmetrically deformed IOL 100 via the plunger 12 acts a counteracting force lager than the other protrusion on the plunger 12. In this case, too, the injector 1 can realize the firm centering of the push member and stabilize the folded position of the IOL.

## Claims

1. An intraocular lens injection instrument (1) for injecting an intraocular lens (100) into a patient's eye includes:
a cylindrical hollow injection part (20) formed with a passage (20b) of which an inner diameter gradually decreases toward a tip end (20a);
a push member (12) configured to push an outer circumferential portion of the intraocular lens while folding the intraocular lens in the passage of the injection part so that the folded intraocular lens is pushed out of the injection part from the tip end of the injection part;
a guide portion (36) for guiding a push-out direction of the push member;
a first protrusion (38) constituting one side wall surface of the guide portion; and
a second protrusion (40) constituting the other side wall surface of the guide portion,
wherein the intraocular lens to be injected by the intraocular lens injection instrument includes a disk-like optical part (110) and at least one support part (112) extending outwardly from an outer circumferential portion (110a) of the optical part,
the intraocular lens includes a rear support part (112B), which is the support part to be placed on a side close to the push member in pushing out the intraocular lens by the pushing member, the rear support part has a distal end portion (118B) and a root portion (116B), the rear support part is folded inwardly in the outer circumferential portion of the optical part, and
the first protrusion (38) and the second protrusion (40) are shaped asymmetrically, **characterized in that**
the first protrusion (38) and the second protrusion (40) are constructed such that, in pushing out the intraocular lens by the pushing member (12), the distal end portion (118B) is positioned on a side close to the first protrusion (38) and the root portion (116B) is positioned on a side close to the second protrusion (40).

2. The intraocular lens injection instrument according to claim 1, wherein a protruding amount (H1) of an end portion (38a) of the first protrusion on a side close to the tip end of the injection part is larger than a protruding amount (H2) of an end portion (40a) of the second protrusion on a side close to the tip end of the injection part.

3. The intraocular lens injection instrument according to claim 2, wherein the end portion of the second protrusion on a side close to the tip end of the injection part is formed so as to be in a portion where the root portion of the rear support part is positioned when the optical part is folded in pushing out the intraocular lens by the push member.

4. The intraocular lens injection instrument according to any one of claims 1 to 3, wherein the second protrusion is formed shorter than the first protrusion in the push-out direction of the push member.

5. The intraocular lens injection instrument according to any one of claims 1 to 4, wherein the guide portion, the first protrusion, and the second protrusion are formed in a cover member (30) constituting a part of the injection part.

## Patentansprüche

1. Einspritzinstrument (1) für eine Intraokularlinse zum Einspritzen einer Intraokularlinse (100) in das Auge eines Patienten, aufweisend:
ein hohles, zylindrisches Einspritzteil (20), das mit einem Durchgang (20b) ausgebildet ist, bei dem ein innerer Durchmesser schrittweise zu einem Spitzenende (20a) hin abnimmt;
ein Schiebeelement (12), das dafür ausgebildet ist, einen äußeren Umfangsabschnitt der Intraokularlinse zu schieben, während die Intraokularlinse in dem Durchgang des Einspritzteils gefaltet wird, so dass die gefaltete Intraokularlinse aus dem Einspritzteil von dem Spitzenende des Einspritzteils ausgeschoben wird;
einen Führungsabschnitt (36) zum Führen einer Ausschieberichtung des Schiebeelements;
einen ersten Vorsprung (38), der eine erste Seitenwandoberfläche des Führungsabschnitts bildet; und
einen zweiten Vorsprung (40), der die andere Seitenwandoberfläche des Führungsabschnitts bildet,
wobei die von dem Einspritzinstrument für eine Intraokularlinse einzuschiebende Intraokularlinse ein scheibenartiges optisches Teil (110) und mindestens ein Halteteil (112) aufweist, das sich von einem äußeren Umfangsabschnitt (110a) des optischen Teils nach außen erstreckt,
wobei die Intraokularlinse ein hinteres Halteteil (112B) aufweist, das das Halteteil ist, das bei dem Ausschieben der Intraokularlinse durch das Schiebeelement an einer Seite in der Nähe des Schiebeelements anzuordnen ist, wobei das hintere Halteteil einen distalen Endabschnitt (118B) und einen Grundabschnitt (116B) aufweist, wobei das hintere Halteteil in dem äußeren Umfangsabschnitt des optischen Teils nach innen gefaltet ist, und
wobei der erste Vorsprung (38) und der zweite Vorsprung (40) asymmetrisch geformt sind,
**dadurch gekennzeichnet, dass**
der erste Vorsprung (38) und der zweite Vorsprung (40) derart ausgebildet sind, dass bei einem Ausschieben der Intraokularlinse durch das Schiebeelement (12) der distale Endabschnitt (118B) an einer Seite in der Nähe des ersten Vorsprungs (38) angeordnet ist und der Grundabschnitt (116B) an einer Seite in der Nähe des zweiten Vorsprungs (40) angeordnet ist.

2. Einspritzinstrument für eine Intraokularlinse nach Anspruch 1, wobei ein vorstehender Teil (H1) eines Endabschnitts (38a) des ersten Vorsprungs an einer Seite in der Nähe des Spitzenendes des Einspritzteils größer ist als ein vorstehender Teil (H2) eines Endabschnitts (40a) des zweiten Vorsprungs an einer Seite in der Nähe des Spitzenendes des Einspritzteils.

3. Einspritzinstrument für eine Intraokularlinse nach Anspruch 2, wobei der Endabschnitt des zweiten Vorsprungs an einer Seite in der Nähe des Spitzenendes des Einspritzteils so ausgebildet ist, dass er in einem Abschnitt liegt, in dem der Grundabschnitt des hinteren Halteteils angeordnet ist, wenn das optische Teil bei einem Ausschieben der Intraokularlinse durch das Schiebeelement gefaltet ist.

4. Einspritzinstrument für eine Intraokularlinse nach einem der Ansprüche 1 bis 3, wobei der zweite Vorsprung kürzer ausgebildet ist als der erste Vorsprung in der Ausschieberichtung des Schiebeelements.

5. Einspritzinstrument für eine Intraokularlinse nach einem der Ansprüche 1 bis 4, wobei der Führungsabschnitt, der erste Vorsprung und der zweite Vorsprung in einem Abdeckelement (30) ausgebildet sind, das ein Teil des Einspritzteils bildet.

## Revendications

1. Instrument d'injection de lentille intra-oculaire (1) destiné à injecter une lentille intra-oculaire (100) dans l'oeil d'un patient comportant :
une partie d'injection cylindrique creuse (20) formée d'un passage (20b) dont un diamètre interne diminue progressivement vers une extrémité en pointe (20a) ;
un élément de poussée (12) configuré de manière à pousser une partie circonférentielle externe de la lentille intra-oculaire tout en pliant la lentille intra-oculaire dans le passage de la partie d'injection de telle sorte que la lentille intra-oculaire pliée est poussée hors de la partie d'injection à partir de l'extrémité en pointe de la partie d'injection ;
une partie de guidage (36) destinée à guider une direction d'expulsion de l'élément de poussée ;
une première saillie (38) constituant une première surface de paroi latérale de la partie de guidage ; et
une seconde saillie (40) constituant l'autre surface de paroi latérale de la partie de guidage,
dans lequel la lentille intra-oculaire à injecter par l'instrument d'injection de lentille intra-oculaire comporte une partie optique en forme de disque (110) et au moins une partie de support (112) s'étendant vers l'extérieur à partir d'une partie circonférentielle externe (110a) de la partie optique,
la lentille intra-oculaire comporte une partie de support arrière (112B), qui est la partie de support à placer sur un côté proche de l'élément de poussée lors de l'expulsion de la lentille intra-oculaire par l'élément de poussée, la partie de support arrière présente une partie d'extrémité distale (118B) et une partie de racine (116B), la partie de support arrière est pliée vers l'intérieur dans la partie circonférentielle externe de la partie optique, et
la première saillie (38) et la seconde saillie (40) sont formées de manière asymétrique, **caractérisé en ce que**
la première saillie (38) et la seconde saillie (40) sont construites de telle sorte que, lors de l'expulsion de la lentille intra-oculaire par l'élément de poussée (12), la partie d'extrémité distale (118B) est positionnée sur un côté proche de la première saillie (38) et la partie de racine (116B) est positionnée sur un côté proche de la seconde saillie (40).

2. Instrument d'injection de lentille intra-oculaire selon la revendication 1, dans lequel une valeur de saillie (H1) d'une partie d'extrémité (38a) de la première saillie sur un côté proche de l'extrémité en pointe de la partie d'injection est supérieure à une valeur de saillie (H2) d'une partie d'extrémité (40a) de la seconde saillie sur un côté proche de l'extrémité en pointe de la partie d'injection.

3. Instrument d'injection de lentille intra-oculaire selon la revendication 2, dans lequel la partie d'extrémité de la seconde saillie sur un côté proche de l'extrémité en pointe de la partie d'injection est formée de manière à être située dans une partie dans laquelle la partie de racine de la partie de support arrière est positionnée lorsque la partie optique est pliée lors de l'expulsion de la lentille intra-oculaire par l'élément de poussée.

4. Instrument d'injection de lentille intra-oculaire selon l'une quelconque des revendications 1 à 3, dans lequel la seconde saillie est formée d'une taille plus faible que la première saillie dans la direction d'extraction de l'élément de poussée.

5. Instrument d'injection de lentille intra-oculaire selon l'une quelconque des revendications 1 à 4, dans lequel la partie de guidage, la première saillie et la seconde saillie sont formées sur un élément de couvercle (30) constituant une partie de la partie d'injection.
